# EUROPÄISCHE PATENTSCHRIFT

(11) **EP 0 557 247 B1**
(45) Veröffentlichungstag und Bekanntmachung des Hinweises auf die Patenterteilung: **26.03.1997**
(21) Anmeldenummer: 93810090.6
(22) Anmeldetag: 12.02.1993
(51) Int. Cl.: D06P 1/642

(54) **Verfahren zur photochemischen und thermischen Stabilisierung von ungefärbten und gefärbten oder bedruckten Polyesterfasermaterialien**
Process for photochemical or thermal stabilisation of dyed and undyed or printed polyester fibrous materials
Procédé de stabilisation photochimique et thermique de matériaux fibreux polyesters teints, non-teints ou imprimés

(30) Priorität: 21.02.1992 CH 541/92
(43) Veröffentlichungstag der Anmeldung: 25.08.1993
(73) Patentinhaber: Ciba Specialty Chemicals Holding Inc., 4057 Basel (CH)
(72) Erfinder: Burdeska, Kurt, Dr., CH-4058 Basel (CH); Reinert, Gerhard, Dr., CH-4123 Allschwil (CH); Reinehr, Dieter, Dr., W-7842 Kandern (DE)

(56) Entgegenhaltungen:
- EP-A- 0 395 938
- FR-A- 1 386 624
- CHEMICAL ABSTRACTS, vol. 77, no. 4, 24. Juli 1972, Columbus, Ohio, US; abstract no. 21432, KUSUSE et al. 'Triazine antistatiic agents for synthetic fibres', Seite 107

## Beschreibung

Die vorliegende Erfindung betrifft neue Hydroxyphenyl-1,3,5-triazinverbindungen und deren Verwendung im Verfahren zur photochemischen und thermischen Stabilisierung von ungefärbten und gefärbten oder bedruckten Polyesterfasermaterialien.

Aus FR-A-1.386.624 sind Aryl-1,3,5-triazine, welche sich von den erfindungsgemässen Hydroxyphenyl-1,3,5-triazinverbindungen durch die Substituenten am Triazinring unterscheiden, und ihre Verwendung zur Verbesserung der Lichtechtheit von organischen Materialien bekannt.

Gefärbte oder bedruckte Polyesterfasermaterialien werden unter Lichteinfluss und insbesondere bei gleichzeitiger Wärmeeinwirkung geschädigt. Für den Einsatz im Automotive-Sektor beispielsweise ist ein wirkungsvoller Schutz von ungefärbten und gefärbten oder bedruckten Fasermaterialien vor UV-Strahlung unerläßlich.

Gegenstand der vorliegenden Erfindung sind demnach Hydroxyphenyl-1,3,5-triazinverbindungen der Formel 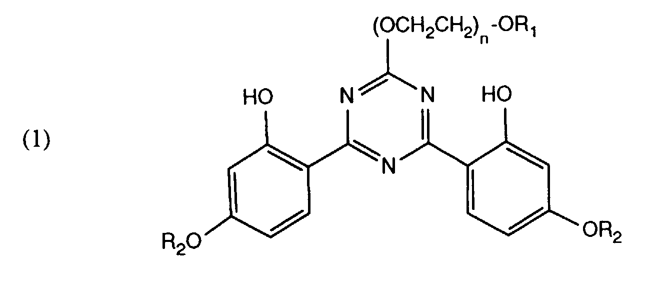 worin
- R₁: C₁-C₄-Ayk,
- R₂: C₁-C₄-Alkyl und
- n: 1 bis 5 bedeuten.

C₁-C₄-Alkyl sind geradkettige oder verzweigte Kohlenwasserstoffreste wie Methyl, Ethyl, n-Propyl, Isopropyl, n-Butyl, sek.Butyl, oder tert.Butyl.

Von besonderem Interesse sind dabei solche Verbindungen, bei denen R₁ und R₂ unabhängig voneinander Methyl oder Ethyl bedeuten.

Ebenso von besonderem Interesse sind dabei solche Verbindungen, bei denen n 1 bis 3 bedeutet.

Von ganz besonderem Interesse sind dabei solche Verbindungen, bei denen R₁ und R₂ unabhängig voneinander Methyl oder Ethyl, und n 1 bis 3 bedeuten.

Als Beispiele für Verbindungen der Formel (1) seien genannt:
- 4,6-Bis(2-hydroxy-4-methoxyphenyl)-2-(2-propoxy-ethoxy)-1,3,5-triazin
- 4,6-Bis(2-hydroxy-4-propoxyphenyl)-2-(2-methoxy-ethoxy)-1,3,5-triazin
- 4,6-Bis(2-hydroxy-4-ethoxyphenyl)-2-(2-methoxy-ethoxy)-1,3,5-triazin
- 4,6-Bis(2-hydroxy-4-methoxyphenyl)-2-(2-methoxy-ethoxy)-1,3,5 -triazin
- 4,6-Bis(2-hydroxy-4-methoxyphenyl)-2-(2-ethoxy-ethoxy)-1,3,5-triazin
- 4,6-Bis(2-hydroxy-4-methoxyphenyl)-2-[2-(2-ethoxy-ethoxy)-ethoxy]-1,3,5-triazin
- 4,6-Bis(2-hydroxy-4-ethoxyphenyl)-2-(2-ethoxy-ethoxy)-1,3,5-triazin
- 4,6-Bis(2-hydroxy-4-ethoxyphenyl)-2-[2-(2-ethoxy-ethoxy)-ethoxy]-1,3,5-triazin
- 4,6-Bis(2-hydroxy-4-ethoxyphenyl)-2-(2-ethoxy-2-methylethoxy)-1,3,5-triazin
- 4,6-Bis(2-hydroxy-4-methoxyphenyl)-2-{2-[2-(2-ethoxy)ethoxy]ethoxy}ethoxy-triazin
- 4,6-Bis(2-hydroxy-4-ethoxyphenyl)-2-{2-[2-(2-ethoxy)ethoxy]ethoxy}ethoxy-1,3,5-triazin
- 4,6-Bis(2-hydroxy-4-ethoxyphenyl)-2-butoxy-1,3,5-triazin

Die Herstellung der Verbindungen der Formel (1) geschieht in an sich bekannter Weise durch Umsetzung einer 4,6-Bis(2-hydroxy-4-alkoxyphenyl)-1,3,5-triazinverbindung der Formel 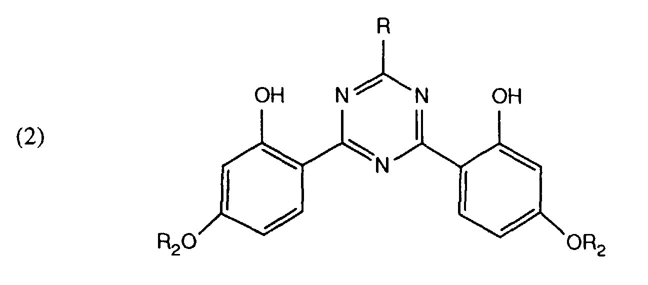 mit einer Verbindung der Formel

(3) M-(OCH₂CH₂)ₙ-OR₁

nach folgendem Schema: 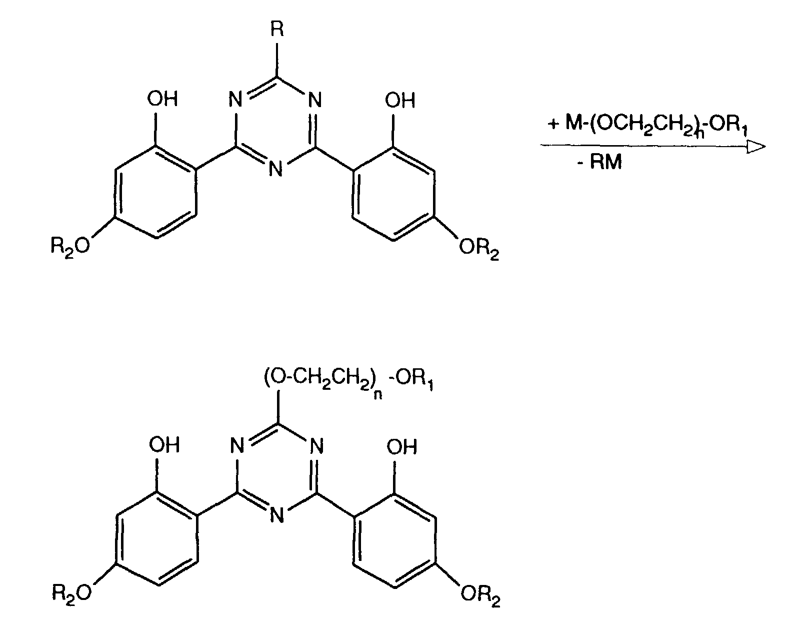 worin
- R: Halogen oder einen Rest der Formel S-R',
- R': C₁-C₄-Alkyl, Phenyl oder Benzyl,
- R₁: C₁-C₄-Alkyl,
- R₂: C₁-C₄-Alkyl,
- M: Alkalimetall und
- n: 1 bis 5 bedeuten.

Vorzugsweise verwendet man Ausgangsverbindungen der Formel (2), bei denen R Thio-C₁-C₄-Alkyl, und insbesondere Thiomethyl bedeutet.

Die Ausgangsverbindungen entsprechend Formel (2) sind bekannt, z.B. aus der CH-A-436,285. Die Herstellung dieser Ausgangsverbindungen kann z.B. analog dem in der EP-A-0,395,938 beschriebenen Verfahren durch Umsetzung von Cyanurchlorid mit C₁-C₄-Alkylmercaptan und der entsprechenden Benzolverbindung erfolgen.

Unter den Verbindungen der Formel (3) sind die Alkalimetallsalze von Alkoholen der allgemeinen Formel

(3a) H-(OCH₂CH₂)ₙ-OR₁

zu verstehen. In Betracht kommen Polyethylenglykolmonoalkylether mit 1 bis 5, vorzugsweise 1 bis 3 Ethylenoxideinheiten. Als Beispiele seien Ethylenglykolmonoalkylether wie Methyl-, Ethyl- oder Propylcellosolve oder Diethylenglykolmonomethyl-, Diethylenglykolmonoethyl-, Diethylenglykolmonopropyl- oder Diethylenglykolmonobutylether oder die entsprechenden Tri-, Tetra- oder Pentaethylenglykolmonoalkylether genannt.

Die Alkohole werden in Form ihrer Lithium-, Kalium- oder Natriumalsalze eingesetzt, wobei die entsprechenden Natriumalkoholate bevorzugt sind.

Einen weiteren Gegenstand der vorliegenden Erfindung stellt das Verfahren zur photochemischen und thermischen Stabilisierung von ungefärbten und gefärbten oder bedruckten Polyesterfasermaterialien, dadurch gekennzeichnet, dass man das Fasermaterial mit der Hydroxyphenyl-1,3,5-triazinverbindung der Formel (1) behandelt.

Die erfindungsgemässen Hydroxyphenyl-1,3,5-triazinverbindungen der Formel (1) werden in einer Menge von 0,5 bis 7,5 Gew.-%, vorzugsweise 0,2 bis 3, und insbesondere 0,5 bis 2 Gew.-% vom Gewicht des Fasermaterials eingesetzt.

Die erfindungsgemässen Hydroxyphenyl-1,3,5-triazinverbindungen der Formel (1) sind in Wasser schwerlöslich und werden daher in dispergierter Form appliziert. Dazu werden sie mit einem entsprechenden Dispergator z.B. mit Hilfe von Quarzkugeln und einem Schnellrührgerät auf eine Feinheit von 1-2 µm gemahlen.

Als Dispergatoren für die Hydroxyphenyl-1,3,5-triazinverbindungen der Fomel (1) kommen in Betracht:
- saure Ester oder deren Salze von Alkylenoxidaddukten, wie z.B. saure Ester oder deren Salze eines Polyadduktes von 4 bis 40 Mol Ethylenoxid an 1 Mol eines Phenols, oder Phosphorsäureester der Addukte von 6 bis 30 Mol Ethylenoxid an 1 Mol 4-Nonylphenol, 1 Mol Dinonylphenol oder besonders an 1 Mol von Verbindungen, die durch Anlagerung von 1 bis 3 Mol von Styrolen an 1 Mol Phenol hergestellt werden,
- Polystyrolsulfonate,
- Fettsäuretauride,
- alkylierte Diphenyloxid-mono- oder -di-sulfonate,
- Sulfonate von Polycarbonsäureestern,
- mit einer organischen Dicarbonsäure, oder einer anorganischen mehrbasischen Säure in einen sauren Ester übergeführte Anlagerungsprodukte von 1 bis 60, vorzugsweise 2 bis 30 Mol Ethylenoxid und/oder Propylenoxid an Fettamine, Fettamide, Fettsäuren oder Fettalkohole mit je 8 bis 22 Kohlenstoffatomen oder an drei- bis sechswertige Alkanole mit 3 bis 6 Kohlenstoffatomen,
- Ligninsulfonate, und ganz besonders
- Formaldehyd-Kondensationsprodukte wie z.B. Kondensationsprodukte von Ligninsulfonaten und/oder Phenol und Formaldehyd, Kondensationsprodukte von Formaldehyd mit aromatischen Sulfonsäuren, wie z. B. Kondensationsprodukte von Ditolylethersulfonaten und Formaldehyd, Kondensationsprodukte von Naphthalinsulfonsäure und/oder Naphthol- oder Naphthylaminsulfonsäuren mit Formaldehyd, Kondensationsprodukte von Phenolsulfonsäuren und/oder sulfoniertem Dihydroxydiphenylsulfon und Phenolen bzw. Kresolen mit Formaldehyd und/oder Harnstoff, sowie Kondensationsprodukte von Diphenyloxid-disulfonsäure-Derivaten mit Formaldehyd.

Als Farbstoffe kommen in Wasser nur gering lösliche Dispersionsfarbstoffe in Betracht. Sie liegen deshalb in der Färbeflotte zum größten Teil in Form einer feinen Dispersion vor. Sie können verschiedenen Farbstoffklassen angehören, beispielsweise den Acridon-, Azo-, Anthrachinon-, Cumarin-, Methin-, Perinon-, Naphthochinonimin-, Chinophthalon-, Styryl-, oder Nitrofarbstoffen. Es können auch Mischungen von Dispersionsfarbstoffen erfindungsgemäss eingesetzt werden.

Als Polyesterfasermaterial, das gefärbt oder bedruckt und mit den genannten Hydroxyphenyl-1,3,5-triazinverbindungen behandelt werden kann, sind z.B. Celluloseesterfasern, wie z.B. Cellulose-2½-acetatfasern und -triacetatfasern und besonders lineare Polyesterfasern, die eventuell auch sauer modifiziert sind, zu verstehen, die z.B. durch Kondensation von Terephthalsäure mit Ethylenglykol oder von Isophthalsäure oder Terephthalsäure mit 1,4-Bis(hydroxymethyl)-cyclohexan erhalten werden, sowie Mischpolymere aus Terephthal- und Isophthalsäure und Ethylenglykol. Das in der Industrie bisher fast ausschließlich eingesetzte lineare Polyesterfasermaterial besteht aus Terephthalsäure und Ethylenglykol.

Die Fasermaterialien können auch als Mischgewebe untereinander oder mit anderen Fasern verwendet werden, wie z.B. Mischungen aus Polyacrylnitril/Polyester, Polyamid/Polyester, Polyester/Baumwolle, Polyester/Viskose und Polyester/Wolle, und nach bekannten Verfahren diskontinuierlich oder kontinuierlich gefärbt oder auch bedruckt werden.

Das Textilmaterial kann in verschiedenen Aufmachungsformen vorliegen. Vorzugsweise kommt Stückware, wie Gewirke oder Gewebe oder auch Garn auf Kreuzspulen, Kettbäumen usw. in Betracht.

Die Färbungen erfolgen aus wässriger Flotte nach einem kontinuierlichen oder diskontinuierlichen Verfahren. Beim diskontinuierlichen Verfahren kann das Flottenverhältnis in einem weiten Bereich gewählt werden, z.B. 1:4 bis 1:100, vorzugsweise 1:6 bis 1:50. Die Temperatur, bei der gefärbt wird, beträgt mindestens 50°C und in der Regel ist sie nicht höher als 140°C. Vorzugsweise liegt sie im Bereich von 80 bis 135°C.

Bei kontinuierlichen Färbeverfahren werden die Färbeflotten, die neben den Farbstoffen gegebenenfalls weitere Hilfsmittel enthalten können, auf das Stückmaterial durch beispielsweise Foulardieren oder Pflatschen aufgebracht und mittels Thermofixier- oder HT-Dämpfprozessen entwickelt.

Lineare Polyesterfasern und Cellulosefasern färbt man vorzugsweise nach dem sogenannten Hochtemperaturverfahren in geschlossenen und druckbeständigen Apparaten bei Temperaturen >100°C, bevorzugt zwischen 110° und 135°C und gegebenenfalls unter Druck. Als geschlossene Gefäße eignen sich beispielsweise Zirkulationsapparaturen wie Kreuzspul- oder Baumfärbeapparate, Haspelkufen, Düsen- oder Trommelfärbemaschinen, Muff-Färbeapparate, Paddeln oder Jigger.

Zum kontinuierlichen Färben werden Foulard oder Pflatschwerke eingesetzt, die Entwicklung erfolgt durch Heissluft im Spannrahmen oder in HT-Dämpfern.

Cellulose-2½-acetatfasern färbt man vorzugsweise bei Temperaturen von 80-85°C.

Werden die erfindungsgemässen Hydroxyphenyl-1,3,5-triazinverbindungen in der Färbeapplikation eingesetzt, so erfolgt die Anwendung so, dass man das Fasermaterial zunächst mit diesen Verbindungen behandelt und anschliessend die Färbung durchführt oder vorzugsweise gleichzeitig das Fasermaterial mit der Hydroxyphenyl-1,3,5-triazinverbindung und dem Farbstoff im Färbebad behandelt. Die Applikation der Hydroxyphenyl-1,3,5-triazinverbindung kann jedoch auch nachträglich auf die fertig hergestellte Färbung mittels Thermofixierung, z.B. bei 190 bis 230°C in einem Zeitraum von 30 Sekunden bis 5 Minuten erfolgen.

Die Färbeflotten können auch weitere Zusätze, wie z.B. Färbereihilfsmittel, Dispergiermittel, Carrier, Wollschutz- und Netzmittel sowie auch Entschäumer enthalten.

Die Färbebäder können desweiteren Mineralsäuren, wie z.B. Schwefelsäure oder Phosphorsäure, oder zweckmässigerweise organische Säuren, zum Beispiel aliphatische Carbonsäuren wie Ameisensäure, Essigsäure, Oxalsäure oder Zitronensäure und/oder Salze wie Ammoniumacetat, Ammoniumsulfat oder Natriumacetat enthalten. Die Säuren dienen vor allem der Einstellung des pH-Wertes der erfindungsgemäss verwendeten Flotten, der zwischen 4 und 5 liegt.

Vorzugsweise lässt man das Fasermaterial während 5 Minuten bei 40 bis 80°C im Bad, das den Farbstoff, die Hydroxyphenyl-1,3,5-triazinverbindung und gegebenenfalls weitere Zusätze enthält und auf einen pH-Wert von 4,5 bis 5,5 eingestellt ist, vorlaufen, erhöht die Temperatur innerhalb von 10 bis 20 Minuten auf 125 bis 130°C und behandelt für 15 bis 90 Minuten, vorzugsweise 30 Minuten, bei dieser Temperatur weiter.

Die Fertigstellung der Färbungen erfolgt durch Abkühlen der Färbeflotte auf 50 bis 80°C, Spülen der Färbungen mit Wasser und gegebenenfalls durch Reinigung auf übliche Weise im alkalischen Medium unter reduktiven Bedingungen. Die Färbungen werden dann wiederum gespült und getrocknet. Bei Verwendung von Küpenfarbstoffen für den Celluloseanteil wird die Ware auf übliche Weise zuerst mit Hydrosulfit bei einem pH-Wert von 6 bis 12,5 und dann mit einem Oxidationsmittel behandelt und schließlich ausgewaschen.

Für die Herstellung von Drucken werden die erfindungsgemässen Hydroxyphenyl-1,3,5-triazinverbindungen in Form ihrer wässrigen Dispersionen den Druckpasten beigemischt. Die Druckpaste enthält dabei die entsprechende Hydroxyphenyl-1,3,5-triazinverbindung in Mengen von 0,5 bis 5 %, vorzugsweise 1 bis 2%, bezogen auf das Gewicht der Druckpaste.

Die Menge der Farbstoffe, die den Druckpasten zugesetzt werden, richtet sich nach der gewünschten Farbnuance; im allgemeinen haben sich Mengen von 0,01 bis 15, vorzugsweise 0,02 bis 10 Gewichtsprozent, bezogen auf das eingesetzte Textilmaterial, bewährt.

Die Druckpasten enthalten neben den Farbstoffen und der wässrigen Hydroxyphenyl-1,3,5-triazinverbindung-Dispersion zweckmässigerweise säurestabile Verdickungsmittel, vorzugsweise natürlicher Herkunft wie Kernmehlabkömmlinge, insbesondere Natriumalginat für sich allein oder im Gemisch mit modifizierter Cellulose, insbesondere mit vorzugsweise 20 bis 25 Gewichtsprozent Carboxymethylcellulose. Daneben können die Druckpasten noch Säurespender wie Butyrolacton oder Natriumhydrogenphosphat, Konservierungsmittel, Sequestriermittel, Emulgatoren, wasserunlösliche Lösungsmittel, Oxidationsmittel oder Entlüftungsmittel enthalten.

In Betracht kommen als Konservierungsmittel vor allem Formaldehyd abgebende Mittel, wie z.B. Paraformaldehyd oder Trioxan, vor allem wässrige, etwa 30 bis 40-gewichtsprozentige Formaldehydlösungen, als Sequestriermittel z.B. nitrilotriessigsaures Natrium, ethylendiamintetraessigsaures Natrium, vor allem Natrium-Polymethaphosphat, insbesondere Natrium-Hexamethaphosphat, als Emulgatoren vor allem Addukte aus einem Alkylenoxid und einem Fettalkohol, insbesondere einem Addukt aus Oleylalkohol und Ethylenoxid, als wasserunlösliche Lösungsmittel hochsiedende, gesättigte Kohlenwasserstoffe, vor allem Paraffine mit einem Siedebereich von etwa 160 bis 210°C (sogenannte Lackbenzine), als Oxidationsmittel z.B. eine aromatische Nitroverbindung, vor allem eine aromatische Mono- oder Dinitrocarbonsäure oder -sulfonsäure, die gegebenenfalls als Alkylenoxidaddukt vorliegt, insbesondere eine Nitrobenzolsulfonsäure und als Entlüftungsmittel z.B. hochsiedende Lösungsmittel, vor allem Terpentinöle, höhere Alkohole, vorzugsweise C₈- bis C₁₀-ASkohole, Terpenalkohole oder Entlüftungsmittel auf Basis von Mineral- und/oder Silikonölen, insbesondere Handelsformulierungen aus etwa 15 bis 25 Gewichtsprozent eines Mineral- und Silikonölgemisches und etwa 75 bis 85 Gewichtsprozent eines C₈-Alkohols wie z.B. 2-Ethyl-n-hexanol.

Beim Bedrucken der Fasermaterialien wird die Druckpaste ganzflächig oder stellenweise direkt auf das Fasermaterial aufgebracht, wobei zweckmässigerweise Druckmaschinen üblicher Bauart, z.B. Tiefdruck-, Rotationssiebdruck- und Flachfilmdruckmaschinen eingesetzt werden.

Das Fasermaterial wird nach dem Bedrucken bei Temperaturen bis 150°C, vorzugsweise 80° bis 120°C getrocknet.

Anschliessend erfolgt die Fixierung durch eine Wärmebehandlung des Materials bei Temperaturen von vorzugsweise 100° bis 220°C. Die Wärmebehandlung erfolgt im allgemeinen mit überhitztem Wasserdampf unter atmosphärischem Druck.

Je nach Temperatur kann die Fixierung 20 Sekunden bis 10 Minuten, vorzugsweise 4 bis 8 Minuten erfolgen.

Die Fertigstellung der Drucke erfolgt ebenfalls auf übliche Weise durch Spülen mit Wasser und kann gegebenenfalls durch zusätzliche Reinigung im alkalischen Medium unter reduktiven Bedingungen, z.B. mittels Natriumdithionit vorgenommen werden. Im letzteren Fall werden die Druckfärbungen wiederum gespült, entwässert und getrocknet.

Mit dem erfindungsgemässen Verfahren lassen sich hochlichtechte und sublimationsbeständige Polyester-Färbungen und Drucke erzielen. Eine gezielte Vor- oder Nachbehandlung des Fasermaterials ist mit dem erfindungsgemässen Verfahren nicht erforderlich.

In den folgenden Herstellungsverfahren und Applikationsbeispielen beziehen sich die Prozentsätze auf das Gewicht. Die Mengen beziehen sich bei den Farbstoffen und bei den Hydroxyphenyl-1,3,5-triazinverbindungen auf Reinsubstanz.

### Herstellung der Hydroxyphenyl-1,3,5-triazinverbindungen

Beispiel 1: 3,5 g (0,15 Mol) Natrium werden in 180 ml Propylcellosolve gelöst. Unter Rühren werden 18,6 g (0,05 Mol) 2-Thiomethyl-4,6-bis(2-hydroxy-4-methoxyphenyl)1,3,5-triazin zugegeben und während 4 Stunden bei 83-85°C gerührt. Dann wird die Reaktionsmischung nach dem Abkühlen auf Raumtemperatur auf 200 ml Eiswasser und 170 ml 1n HCl gegossen und abfiltriert. Es wird dann mit Methanol gewaschen und bei 80°C im Vakuumschrank getrocknet. Man erhält 20,6 g eines hellen Produktes der Formel 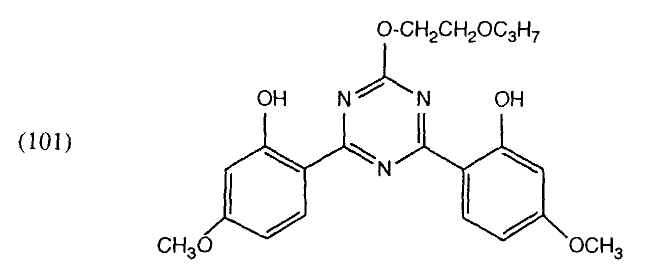
- Ausbeute:: 96,4% d.Th.
- Fp.: 119-120°C
- λₘₐₓ: 296/351 nm

| Elementaranalyse: | | | |
|---|---|---|---|
| Gefunden: | 61,70%C; | 5,9% H; | 9,9%N |
| Berechnet als C₂₂H₂₅N₃O₆: | 61,82%C; | 5,89%H; | 9,83%N; |

Beispiele 2 bis 14: Nach dem in Beispiel 1 beschriebenen Verfahren werden die nachfolgenden Verbindungen (102) bis (111) hergestellt. (Tabelle 1)

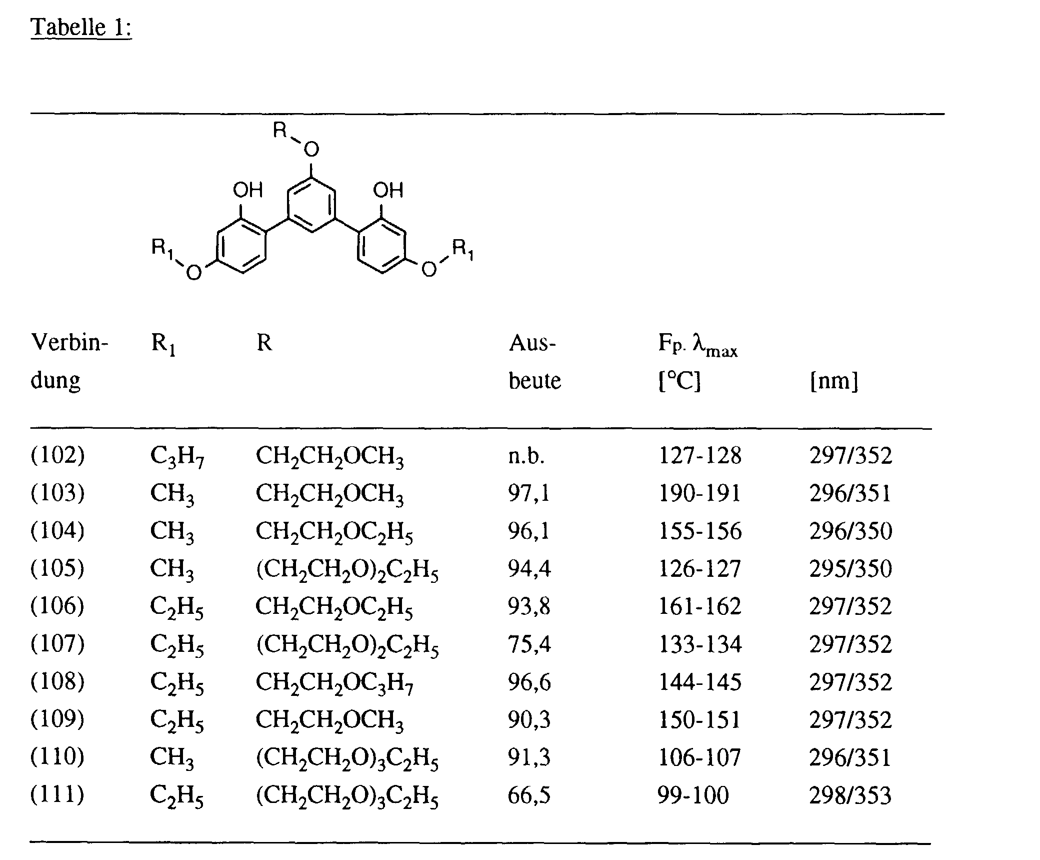

### Applikationsbeispiele

### Beispiele 15 bis 29: Anwendung in der Färberei

Es werden 15 Muster von je 10 g eines PES-Trikots in einem HT-Färbeapparat, z.B. einem ®Turbomat (Firma Mathis, Niederhasli) bei einem Flottenverhältnis von 1:10 gefärbt. Die Flotten enthalten 2g/l Ammoniumsulfat, 0,5 g/l eines Färbereihilfsmittels, z.B. ®Univadin 3-flex und die Farbstoffe der Formeln (I) bis (IV) in folgenden Mengen: 0,05% des Farbstoffs der Formel 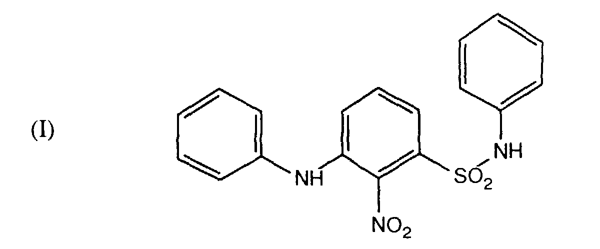 0,025% des Farbstoffs der Formel 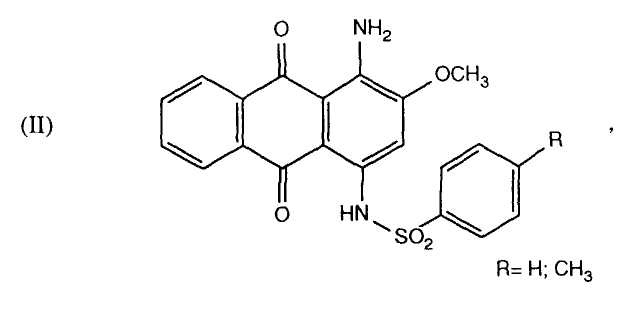 0,03% des Farbstoffes der Formel 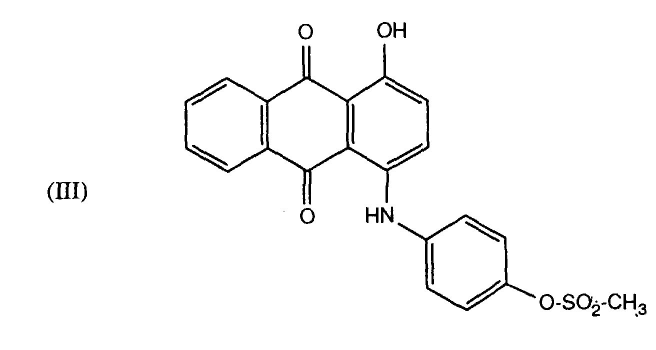 und 0,025% des Farbstoffes der Formel

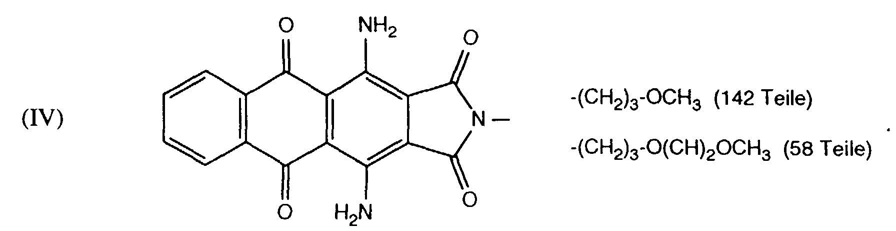

Während die Flotte 1 keine weiteren Zusätze enthält, werden den Flotten 2 bis 15 zusätzlich noch jeweils 2% der formulierten Verbindungen (101) bis (111) zugesetzt.

Die Hydroxyphenyl-1,3,5-triazinverbindungen werden vor dem Einsatz in der Färbeflotte oder als Druckpaste formuliert. Dazu werden
- die jeweiligen Verbindungen,
- das als Dispergator eingesetzte Kondensationsprodukt aus Naphthalinsulfonsäure und Formaldehyd im Verhältnis 1:1,
- die 2-4 fache Menge Wasser und
- die vierfache Menge Quarzkugeln (⌀ 1mm)
mit einem Schnellrührer so lange gemahlen, bis das Produkt eine Feinheit von 1-2 µm aufweist. Die Dispersion wird mit einem feinen Maschensieb abgetrennt und mit 0,5% Carboxymethylcellulose stabilisiert und auf 30% eingestellt.

Die Trikotstücke werden in den dispergierten Flotten in Druckbomben gefärbt. Dazu wird bei 70°C eingegangen und nach einer Behandlungszeit von 5 Minuten innerhalb von 10 Minuten auf 100°C und schließlich innerhalb von 20 Minuten auf 130°C erhitzt. Bei dieser Temperatur färbt man 30 Minuten, kühlt auf 50°C ab und spült warm und kalt. Es folgt eine reduktive Nachreinigung bei 70°C, die innerhalb von 30 Minuten mit 2 ml/l Natronlauge 36 Bé und 3 g/l Natriumdithionit durchgeführt wird. Danach wird erneut warm und kalt gespült, zentrifugiert und getrocknet.

Zur Ermittlung der Lichtechtheiten werden die Färbungen nach DIN 75.202 (FAKRA) und nach SAE J 1885 belichtet. Die Ergebnisse sind in Tabelle 2 aufgeführt.

**Tabelle 2:**

| Färbung (Flotte Nr.) | Lichtechtheiten nach | | |
|---|---|---|---|
| | FAKRA 3 Cyclen | FAKRA 5 cyclen | SAE 420 KJ |
| ohne Zusatz | 2-3 | 2 H | 1-2 |
| (1) + Verbindung (101) | 4 | 3-4 H | 3 H |
| (2) + Verbindung (102) | 4 | 3-4 H | 3 H |
| (3) + Verbindung (103) | 4 | 3-4 H | 3 H |
| (4) + Verbindung (104) | 4 | 3-4 H | 3 H |
| (5) + Verbindung (105) | 4 | 3-4 H | 3 H |
| (6) + Verbindung (106) | 4 | 3-4 H | 3 H |
| (7) + Verbindung (107) | 4 | 3-4 H | 3 H |
| (8) + Verbindung (108) | 4 | 3-4 H | 3 H |
| (9) + Verbindung (109) | 4 | 3-4 H | 3 H |
| (10) + Verbindung (110) | 3-4 | 3 H | 2-3 |
| (11) + Verbindung (111) | 3-4 | 3 H | 2-3 |

Aus Tabelle 2 ist ersichtlich, dass die Hellgraufärbungen bei Verwendung der Hydroxyphenyl-1,3,5-triazinverbindungen (101)-(111) hinsichtlich ihrer Lichtechtheitseigenschaften deutlich verbessert werden.

### Beispiele 30 bis 35: Anwendung im Druck

Zur Bedruckung von PES-Autopolsterstoff werden Druckpasten mit folgender Zusammensetzung verwendet:
750 Teile einer Stammverdickung enthaltend
- 9: Teile Stärkeether als Verdickungsmittel
- 18: Teile Natriumalginat als Verdickungsmittel
- 3,75: Teile Natriumdihydrogenphosphat
- 2,48: Teile Natriumchlorat und
- 716,77: Teile Wasser.

Diese Stammverdickung wird vermischt mit
- 6,4: Teilen des Farbstoffgemisches bestehend aus
- 2,0: Teilen des Farbstoffes der Formel 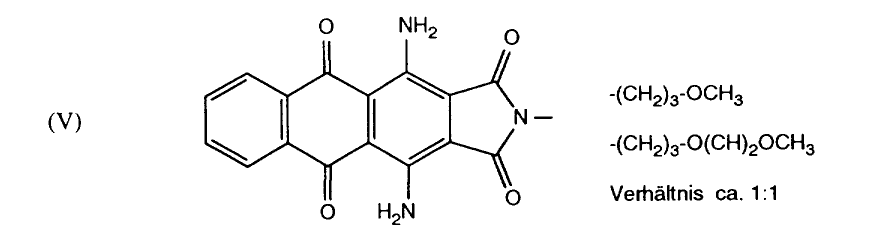
- 1,4: Teilen des Farbstoffes der Formel 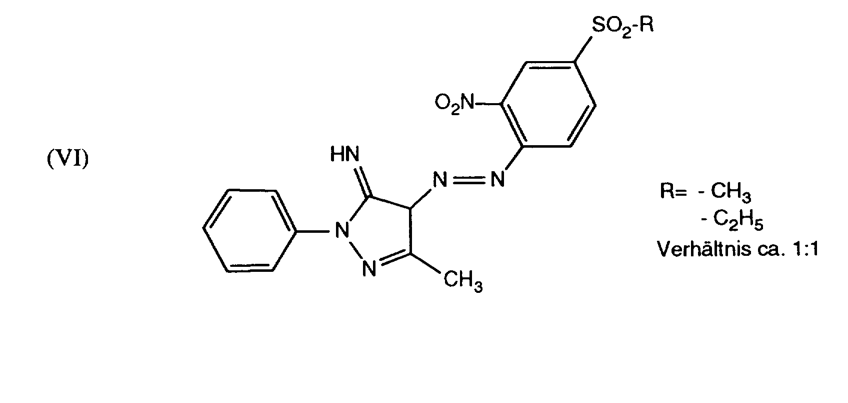
- 2,0: Teilen des Farbstoffes der Formel 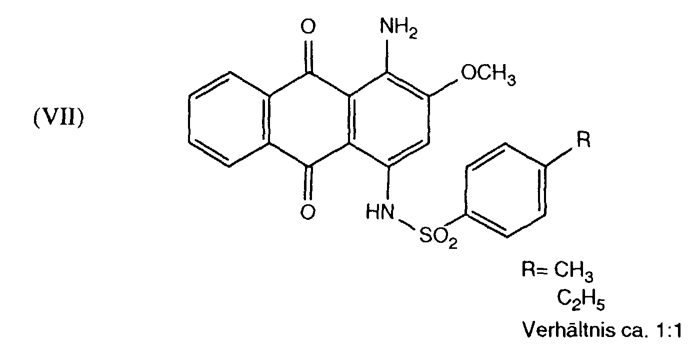 und
- 1,0: Teilen des Farbstoffes der Formel 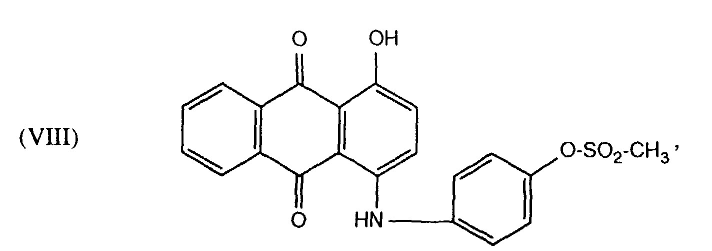
- 213,6: Teilen Wasser und
- 30: Teilen der gemäss Beispiel 15 dispergierten 30%igen Hydroxyphenyl-1,3,5-triazinformulierung enthaltend jeweils die Verbindungen (101), (103) und (109).

Mit diesen Druckpasten werden die vorgereinigten PES-Trikotmuster auf einem Drucktisch nach Zimmer (Hersteller Firma Zimmer, Klagenfurt/Österreich) bedruckt. Diese Muster werden getrocknet und mit überhitztem Dampf bei 180°C während 8 Minuten gedämpft. Danach wird mit kaltem Wasser gespült und bei 70°C 30 Minuten in Bädern mit 2 ml/l Natronlauge 36° Bé und 3 g/l Natriumdithionit reduktiv gereinigt. Abschliessend wird warm und kalt gespült und nach dem Zentrifugieren bei 80°C getrocknet. Die Muster werden auf ihre Lichtechtheiten nach SN-ISO 105 B02 (XENON), nach DIN 75.202 (FAKRA) und nach SAE J 1885 (SAE) geprüft. Die Ergebnisse sind in Tabelle 3 zusammengestellt.

**Tabelle 3:**

| Drucke | Lichtechtheiten nach | | | |
|---|---|---|---|---|
| | XENON 800 h | FAKRA 4 Cyclen | SAE 489 KJ | SAE 600 KJ |
| ohne Zusatz | 7-8 | 1-2 RH | 1 RH | 1 RH |
| (1) + Verbindung (101) | 7-8 | 4 | 3-4 H | 3-4 H |
| (2) + Verbindung (103) | 7-8 | 4 | 3-4 H | 3 H |
| (3) + Verbindung (109) | 8 | 4 | 3-4 H | 3 H |

Aus den Ergebnissen der Tabelle 3 ist ersichtlich, dass die eingesetzten Hydroxyphenyl-1,3,5-triazinverbindungen eine deutliche Verbesserung der Heisslichtechtheiten bewirken.

## Patentansprüche

1. Hydroxyphenyl-1,3,5-triazinverbindung der Formel 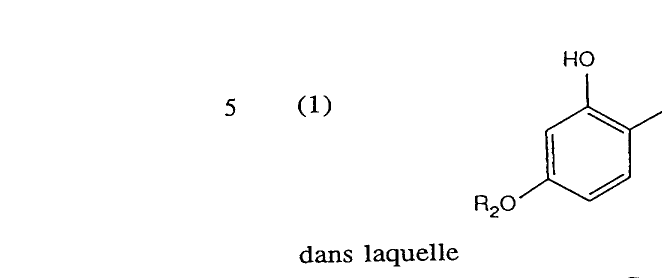 worin
R₁ C₁-C₄-Alky-, R₂ C₁-C₄-Alkyl und n 1 bis 5 bedeutet.

2. Verbindungen gemäss Anspruch 1, dadurch gekennzeichnet, dass R₁ und R₂ unabhängig voneinander Methyl oder Ethyl bedeuten.

3. Verbindungen gemäss einem der Ansprüche 1 oder 2, dadurch gekennzeichnet, dass n 1 bis 3 bedeutet.

4. Verfahren zur Herstellung einer 1,3,5-Triazinverbindung der Formel (1), dadurch gekennzeichnet, dass man eine Triazinverbindung der Formel 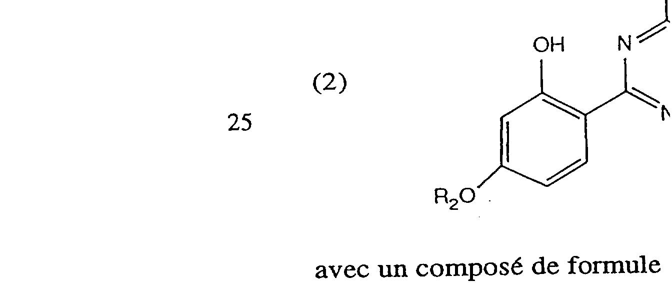 mit einer Verbindung der Formel
(3) M-(OCH₂CH₂)ₙ-OR₁
nach folgendem Schema: 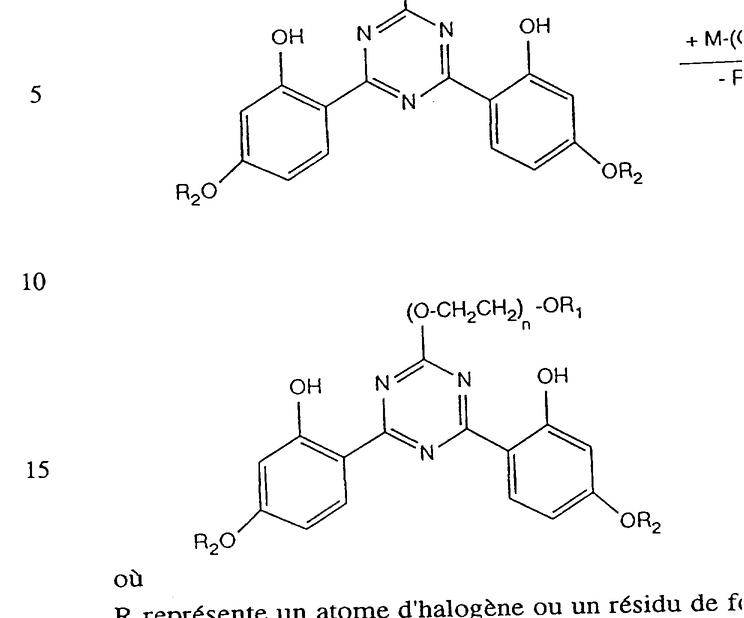 umsetzt, worin
R Halogen oder einen Rest der Formel S-R', R' C₁-C₄-Alkyl, Phenyl oder Benzyl, R₁ C₁-C₄-Alkyl, R₂ C₁-C₄-Alkyl, M Alkalimetall und n 1 bis 5 bedeuten.

5. Verfahren zur photochemischen und thermischen Stabilisierung von ungefärbten und gefärbten oder bedruckten Polyesterfasermaterialien, dadurch gekennzeichnet, dass man das Fasermaterial mit der Hydroxyphenyl-1,3,5-triazinverbindung der Formel (1) gemäss Anspruch 1 behandelt.

6. Verfahren gemäss Anspruch 5, dadurch gekennzeichnet, dass man die Hydroxyphenyl-1,3,5-triazinverbindung der Formel (1) in einer Menge von 0,5 bis 7,5 Gew.-% des Fasermaterials verwendet.

7. Verfahren gemäss einem der Ansprüche 5 oder 6, dadurch gekennzeichnet, dass man die Hydroxyphenyl-1,3,5-triazinverbindung der Formel (1) in der Färbeapplikation direkt dem Färbebad oder der Foulardflotte zusetzt.

8. Verfahren gemäss einem der Ansprüche 5 bis 7, dadurch gekennzeichnet, dass man die Hydroxyphenyl-1,3,5-triazinverbindung der Formel (1) bei der Druckapplikation der Druckpaste beimischt.

## Claims

1. A hydroxyphenyl-1,3,5-triazine compound of formula 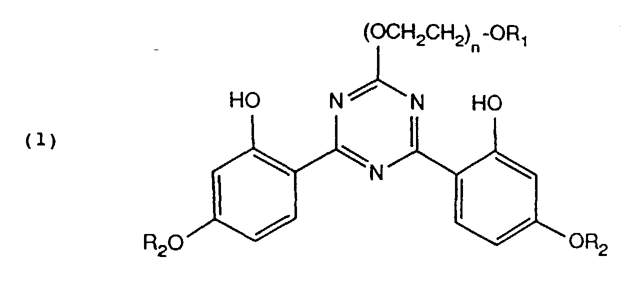 wherein
R₁ is C₁-C₄alkyl, R is C₁-C₄alkyl, and n is 1 to 5.

2. A compound according to claim 1, wherein R₁ and R₂ are each independently of the other methyl or ethyl.

3. A process according to one of claims 1 or 2, wherein n is 1 to 3.

4. A process for the preparation of a 1,3,5-triazine compound of formula (1), which comprises reacting a triazine compound of formula 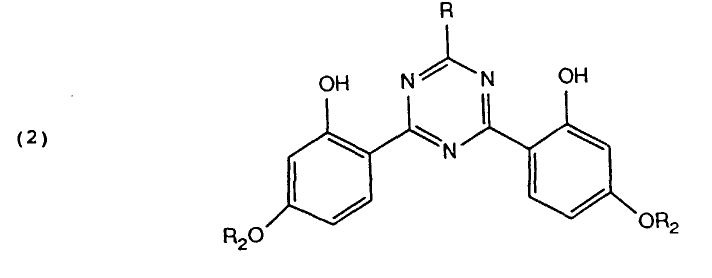 with a compound of formula
(3) M-(OCH₂CH)ₙ-OR₁
according to the following scheme: 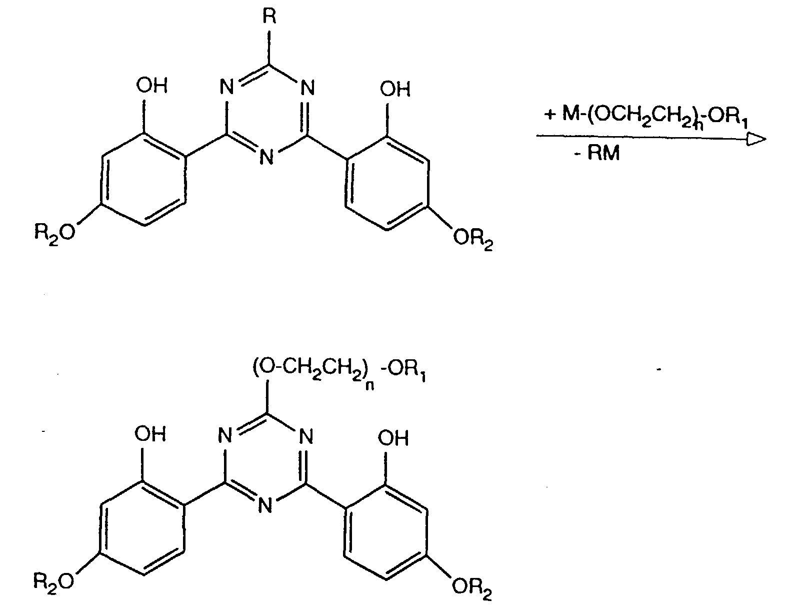 wherein
R is halogen or a radical of formula S-R', R' is C₁-C₄alkyl, phenyl or benzyl, R₁ is C₁-C₄alkyl, R₂ is C₁-C₄alkyl, M is alkali metal, and n is 1 to 5.

5. A process for the photochemical and thermal stabilization of undyed and dyed or printed polyester fibre materials which comprises treating the fibre material with a hydroxyphenyl-1,3,5-triazine compound of formula (1) according to claim 1.

6. A process according to claim 5, wherein a hydroxyphenyl-1,3,5-triazine compound of formula (1) is used in an amount of 0.5 to 7.5 % by weight of the fibre material.

7. A process according to one of claims 5 or 6, wherein the hydroxyphenyl-1,3,5-triazine compound of formula (1) is added directly to the dyebath or to the padding liquor in the dyeing application.

8. A process according to one of claims 5 to 7, wherein the hydroxyphenyl-1,3,5-triazine compound of formula (1) is mixed with the print paste in the printing application.

## Revendications

1. Composé de type hydroxyphényl-1,3,5-triazine de formule 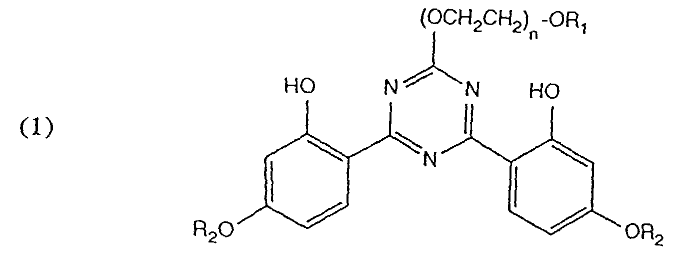 dans laquelle
R₁ est un résidu alkyle en C₁₋₄,
R₂ est un résidu alkyle en C₁₋₄, et
n vaut entre 1 et 5.

2. Composé conforme à la revendication 1, caractérisé en ce que R₁ et R₂ représentent, indépendamment l'un de l'autre, un groupe méthyle ou éthyle.

3. Composé conforme à une des revendications 1 ou 2, caractérisé en ce que n vaut entre 1 et 3.

4. Procédé de préparation d'un composé de type 1,3,5-triazine de formule (1), caractérisé en ce que l'on fait réagir un composé de type triazine de formule 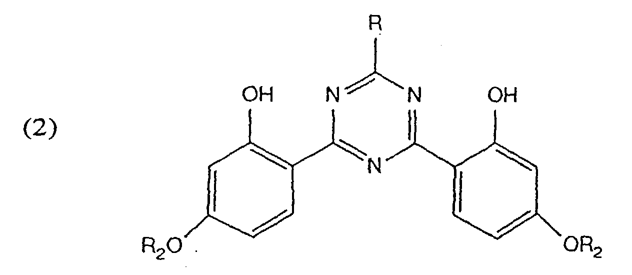 avec un composé de formule
(3) M-(OCH₂CH₂)ₙ-OR₁
selon le schéma réactionnel suivant : 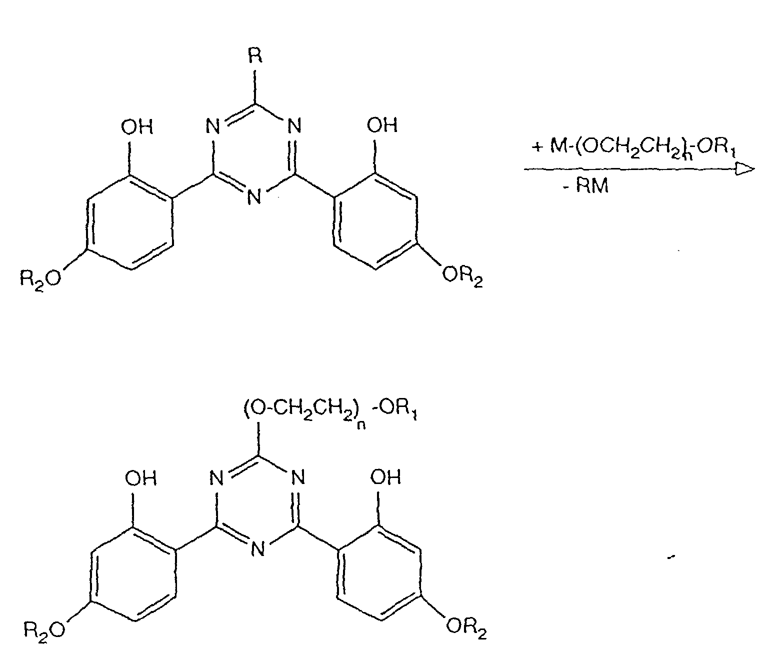 où
R représente un atome d'halogène ou un résidu de formule S-R',
R' est un résidu alkyle en C₁₋₄, phényle ou benzyle,
R₁ est Un résidu alkyle en C₁₋₄,
R₂ est un résidu alkyle en C₁₋₄,
M est un métal alcalin et
n vaut entre 1 et 5.

5. Procédé de stabilisation thermique et photochimique de matériaux fibreux en polyester non teints, teints ou imprimés, caractérisé en ce que l'on traite le matériau fibreux avec le composé de type hydroxyphényl1,3,5-triazine de formule (1) conforme à la revendication 1.

6. Procédé conforme à la revendication 5, caractérisé en ce que l'on utilise le composé de type hydroxyphényl-1,3,5-triazine de formule (1) à raison de 0,5 à 7,5 % en poids rapporté au substrat fibreux.

7. Procédé conforme à une des revendications 5 ou 6, caractérisé en ce que, dans les applications de teinture, on ajoute le composé de type hydroxyphényl-1,3,5-triazine de formule (1) directement au bain de teinture ou au bain de foulardage.

8. Procédé conforme à une des revendications 5 à 7, caractérisé en ce que, lors d'une utilisation pour l'impression, on mélange le composé de type hydroxyphényl-1,3,5-triazine de formule (1) à la pâte d'impression.
